# EUROPEAN PATENT APPLICATION

(11) **EP 3 028 711 A1**
(43) Date of publication of application: **08.06.2016**
(21) Application number: 14196512.9
(22) Date of filing: 05.12.2014
(51) Int. Cl.: A61K 38/14, C07H 15/12, C07K 14/605, C07K 9/00, C07K 14/00, C07K 19/00, A61K 38/00

(54) **Molecular building blocks capable of assembling carbohydrate derivatives, fatty acid residues and peptides**

(71) Applicant: Gubra ApS, 2970 Horsholm (DK)
(72) Inventor: van Witteloostujn, Søren Blok, 2450 København SV (DK); Jensen, Knud Jørgen, 2100 Copenhagen Ø (DK); Jelsing, Jacob, 4000 Roskilde (DK); Vrang, Niels, 2930 Klampenborg (DK); Pedersen, Søren Ljungberg, 4140 Borup (DK)
(74) Representative: COPA Copenhagen Patents

(57) **Abstract**

The present invention relate to molecular building blocks of the formula R₁-A-R₃, wherein A is a carbohydrate derivative and R₁ is hydrogen or an amino protecting group and R₃ is either a hydroxyl, a protecting group or a leaving group. The building blocks are capable of attaching carbohydrate derivatives and fatty acid residues to amino acids. By use of these carbohydrate building blocks, means for providing proteins and peptides with the additional functionalities of carbohydrates and of fatty acid residues and particularly to provide peptides and proteins with the combined functionalities of fatty acids and carbohydrates are provided.

## Description

### FIELD OF THE INVENTION

The present invention relates to molecular building blocks capable of attaching carbohydrate derivatives and fatty acid residues to amino acids, and the use thereof for lipidation of peptide and protein structures. The present invention further relates to peptides comprising carbohydrate derivatives and fatty acid residues, and the use thereof as medicaments.

### BACKGROUND OF THE INVENTION

The use of peptides as medicaments for the treatment of a range of medical conditions is well-known. However, such peptides are often faced with stability problems *in vivo.* Consequently, there is a ubiquitous need for (the possibility of) improving the pharmacokinetics of therapeutic peptides.

In an effort to solve this problem, several therapeutic peptides and proteins have been lipidated, resulting in improved properties in terms of half-life of the peptide *in vivo.* In general, larger fatty acid chains provide better effect in terms of improved stability.

However, the attachment of fatty acids to peptides and proteins also results in decreased solubility of the derivatized peptide, which is not desired. In general, larger fatty acid chains decreases solubility to a greater extent than shorter fatty acid chains.

In an effort to solve these problems, WO 2010/029159 A1 describes the manufacture of derivatized peptides comprising a fatty acid structure and a polyethylene glycol (PEG) structure, where the PEG structure provides an increased hydrophilicity to the lipidated peptides.

However, the use of PEG structures in medicinal peptides is undesired as the biodegradability of such structures is unknown. It is feared that internalized PEG consequently accumulates in the body of the patients, with unknown long-term effects.

Thus, it was an object of the present invention to provide lipidated peptides and proteins with improved solubility, and to provide the means for production of lipidated peptides and proteins with improved solubility.

### SUMMARY OF THE INVENTION

The invention is based on the development of carbohydrate structures capable of forming covalent bonds to amino acids and to fatty acid residues, thereby providing carbohydrate building blocks capable of assembling peptide or protein structures and fatty acid residues, while providing increased solubility.

Thus, the invention relates to agents of the formula R₁-A-R₃, wherein A is a carbohydrate derivative and the agent R₁-A-R₃ is either 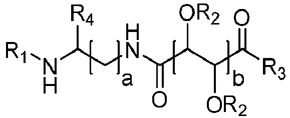 or 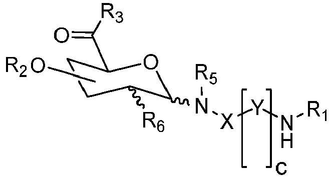 or 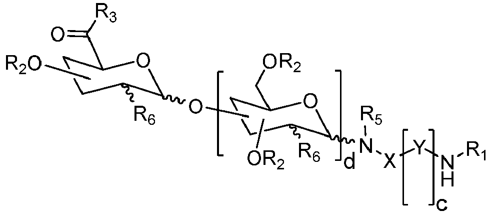 , wherein R₁ is hydrogen or an amino protecting group, or wherein R₁ is a group comprising one or more further groups attached via amide linkage to A,
each R₂ is, individually, either hydrogen or a hydroxyl protecting group,
a is 1, 2, 3, 4, 5 or 6,
b is 1, 2, 3 or 4,
c is 1, 2, 3, 4, 5 or 6,
d is 1, 2, 3, 4, 5, or 6, and
R₃ is either hydroxyl, an O-protecting group, an *O*-leaving group, or wherein R₃ is one or more amino acids,
R₄ is either hydrogen, a hydroxyalkyl with 1-6 carbon atoms, a protected hydroxyalkyl with 1-6 carbon atoms, an alkyl carboxylic acid with 1-6 carbon atoms or a protected alkyl carboxylic acid of 1-6 carbon atoms,
R₅ is hydrogen, methyl, benzyl, alkyl or substituted benzyl,
R₆ is hydrogen, hydroxyl, protected hydroxyl, amine or protected amine,
X is oxygen or nitrogen (NH), and
Y is either -CH₂- or -(CH₂CH₂O)ₙCH₂CH₂-, where n is selected from the group consisting of 1, 2, 3, 4, or 5.

These carbohydrate structures were found to be capable of assembling structures comprising free amines (e.g. an amino acid) with structures comprising carbonyl groups, thereby capable of assembling e.g. peptide or protein structures and fatty acid residues, while providing the functionalities of the carbohydrate structure (e.g. increased water solubility without the potential toxicity problems associated with PEG) to the assembled molecular structures.

### DETAILED DESCRIPTION OF THE INVENTION

The present invention relates to molecular building blocks capable of attaching carbohydrate derivatives and fatty acid residues to amino acids. By use of these carbohydrate building blocks, it was found possible to provide proteins and peptides with the additional functionalities of carbohydrates (e.g. providing increased hydrophilicity) and of fatty acid residues (e.g. providing stability, providing ability to bind to other molecules such as serum albumin) and particularly to provide peptides and proteins with the combined functionalities of fatty acids and carbohydrates.

During the research leading to the present invention, it was found possible to provide such carbohydrate building blocks.

The invention is thus based on the development of a carbohydrate structure having a free amine group (-NH(R₁)) to which other molecular groups, preferably a group comprising one or more further groups B, C, D (defined below) or A (defined above as part of the formulas 1 (a), 1(b) or 1(c))), can be attached by covalent linkage via amide bonds, and having a carboxylic acid or carboxylic acid ester group (-CO(R₃)) to which an amino acid can be attached by covalent linkages via amide bonds.

A preferred aspect of the present invention, was found to be a carbohydrate structure R₁-A-R₃, which consist of the following formula 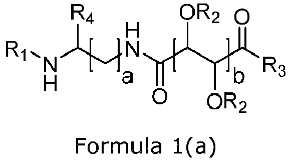 , where a is 1, 2, 3, 4, 5 or 6,
b is 1, 2, 3 or 4, and
the groups R₁, R₂, R₃, and R₄ are as defined below.

Another possible basis of such building blocks, another preferred aspect of the present invention, was found to be a carbohydrate structure R₁-A-R₃, which consist of the following formula 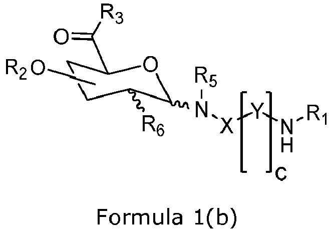 , where c is 1, 2, 3, 4, 5 or 6,
X is oxygen or nitrogen (NH),
Y is either -CH₂- or -(CH₂CH₂O)ₙCH₂CH₂-, where n is selected from the group consisting of 1, 2, 3, 4, or 5, and
the groups R₁, R₂, R₃, R₅, and R₆ are as defined below.

Yet another possible basis of such building blocks, another preferred aspect of the present invention, was found to be a carbohydrate structure R₁-A-R₃, which consist of the following formula 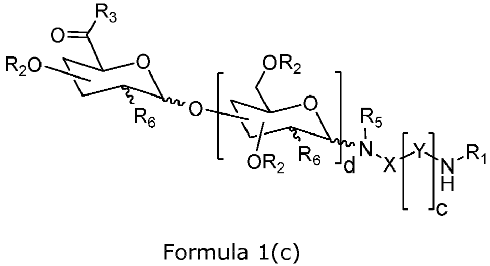 , where c is 1, 2, 3, 4, 5 or 6,
d is 1, 2, 3, 4, 5, or 6,
X is oxygen or nitrogen (NH),
Y is either -(CH₂)ₙ- or -(CH₂CH₂O)ₙCH₂CH₂-, where n is selected from the group consisting of 1, 2, 3, 4, or 5, and
the groups R₁, R₂, R₃, R₅, and R₆ are as defined below.

### The R₁-group:

In a preferred aspect of the invention, the invention relates to a carbohydrate building block (R₁-A-R₃) according to one of the above formulas, i.e. formulas 1(a), 1(b) and 1(c), that contains a free amine or a protected amine (the R₁NH- group, e.g. Boc-NH-or Fmoc-NH-) that is capable of forming amide bonds to a carbonyl group. Thus in a preferred aspect of the invention, the invention relates to carbohydrate agents of the above formulas, i.e. formulas 1(a), 1(b) and 1(c), where R₁ is hydrogen or an amino protecting group.

These carbohydrate building blocks (where the R₃ group can be either a hydroxyl (i.e. -OH), a protecting group (e.g. -*O*-benzyl, -*O*-Si(CH₃)₃ or *-O-tert*-butyl) or a leaving group (e.g. -*O*-NHS (-*O*-(*N*-hydroxysuccinimide)) or -O-Pfp (-*O-*(pentafluorophenyl))), or where the R₃ group may be an amino acid containing molecular group linked via an amide bond to the carbohydrate structure) may be used to attach the carbohydrate structure, or the amino acid containing carbohydrate structure to another molecular structure having a carbonyl group, through an amide linkage.

In another preferred aspect of the invention, the invention relates to a carbohydrate building block (R₁-A-R₃) according to one of the above formulas, i.e. formulas 1(a), 1(b) and 1(c), that contains another molecular group (R₁) linked via an amide bond (the R₁NH- group) to the carbohydrate structure or the amino acid containing carbohydrate structure. Thus in another preferred aspect of the invention, the invention relates to a carbohydrate building block (R₁-A-R₃) according to the above formulas, i.e. formulas 1(a), 1(b) and 1(c), where R₁ is a molecular group linked via an amide bond, preferably a group comprising one or more further groups B, C, D (as defined below) or A (as defined above).

These carbohydrate building blocks (where the R₃ group be either a hydroxyl (i.e. -OH), a protecting group (e.g. -*O*-benzyl, -*O*-Si(CH₃)₃ or *-O-tert*-butyl) or a leaving group (e.g. -*O*-NHS or -*O*-Pfp), or where the R₃ group is an amino acid containing molecular group linked via an amide bond to the carbohydrate structure) may be used to attach the carbohydrate structure to another molecular structure having a free amine, through an amide linkage.

### The R₃-group:

In a preferred aspect of the invention, the invention relates to a carbohydrate building block (R₁-A-R₃) according to one of the above formulas, i.e. formulas 1(a), 1(b) and 1(c), that contains a carboxylic acid or carboxylic acid ester group (the -COR₃ group) that is capable of forming amide bonds with a free amine (e.g. a free amine present in an amino acid, either the *N*-terminal amine or a side-chain amine (such as the epsilon amine of Lys)). Accordingly, in a preferred embodiment the R₃ is either a hydroxyl group (i.e. -OH) or a leaving group (e.g. -*O*-NHS or -*O*-Pfp) which, when reacted with a free amine, facilitates the formation of an amide bond between the carbonyl group and a free amine.

Thus, in a preferred aspect of the invention, the invention relates to carbohydrate building blocks of one of the above formulas, i.e. formulas 1(a), 1(b) and 1(c), where R₃ is either a hydroxyl (i.e. -OH), a protecting group (e.g. -*O*-benzyl, -*O*-Si(CH₃)₃ or *-O-tert*-butyl) or a leaving group (e.g. -*O*-NHS or -*O*-Pfp).

These carbohydrate building blocks (where R₁ is hydrogen or an amino protecting group (e.g. Fmoc or Boc)), or wherein R₁ is a molecular group linked via an amide bond) may be used to attach the carbohydrate structure to another molecular structure having a free amine (e.g. a free amine present in an amino acid, either the *N*-terminal amine or a side-chain amine (such as the epsilon amine of Lys)) through an amide linkage.

In another preferred aspect of the invention, the invention relates to a carbohydrate building block (R₁-A-R₃) according to one of the above formulas, i.e. formulas 1(a), 1(b) and 1(c), that contains an amino acid containing molecular group (R₃) linked via an amide bond (the -COR₃ group) to the carbohydrate structure. Thus in another preferred aspect of the invention, the invention relates to a carbohydrate building block (R₁-A-R₃) according to the above formulas, i.e. formulas 1(a), 1(b) and 1(c), where R₃ is one or more amino acids (D).

The -COR₃-group in the ring-closed building blocks according to the invention:

In the above formulas 1(b) and 1(c) the -COR₃-group may be situated on any of the carbon atom number 3, 4 and 5 of the carbohydrate anomer.

Preferably, however, the -COR₃-group is situated on the carbon atom number 5.

### R₂

According to the present invention, the carbohydrate building block (R₁-A-R₃) according to the above formulas, i.e. formulas 1(a), 1(b) and 1(c), contain an R₂-group which is, individually, either hydrogen or a hydroxyl protecting group (e.g. -*O*-Benzyl, -*O*-Acetyl, -*O*-*tert*-butyl, -O-TBDMS, -*O*-Benzoyl or -*O*-Methyl).

Preferably, R₂ is hydrogen, acetyl (Ac), benzyl (Bn) or benzoyl (Bz).

### The -OR₂-groups in the ring-closed building blocks according to the invention:

In the above formula 1(b) the -OR₂-groups may be situated on any one or each of the carbon atom number 3 and 4 of the carbohydrate anomer. Preferably, however, the -OR₂-group is situated on both the carbon atom number 3 and 4.

In the above formula 1(c) the -OR₂-groups of the carbohydrate anomer bearing the R₃ structure may be situated on any one or each of the carbon atoms 3 and 4 of that carbohydrate anomer. Preferably, however, the -OR₂-group is situated on both the carbon atom number 3 and 4 of that anomer.

The -OR₂-groups of the additional carbohydrate anomer(s) (i.e. the anomers not bearing the -COR₃ structure) may be situated on any one or each of the carbon atom number 3, 4, and 6 of the respective carbohydrate anomer, except the carbon atom to which another carbohydrate anomer is attached. Preferably, the -OR₂-group is situated on the carbon atom number 3 and 6 of that anomer.

### R₄

According to the present invention, the carbohydrate building block (R₁-A-R₃) according to the above formula 1(a), contain an R₄-group which is either hydrogen, a hydroxyalkyl with 1-6 carbon atoms (-(CH₂)ₙOH, where n is 1-6), a protected hydroxyalkyl with 1-6 carbon atoms (-(CH₂)ₙOR₂, where n is 1-6), an alkyl carboxylic acid with 1-6 carbon atoms (-(CH₂)ₙCOOR₇, n is 1-6) or a protected alkyl carboxylic acid of 1-6 carbon atoms (-(CH₂)ₙCOOR₇, n is 1-6), wherein R₇ is either hydrogen or a protecting group (e.g. -*O*-benzyl, -*O*-Si(CH₃)₃ or *-O-tert*-butyl)*.*

Preferably, R₄ is hydrogen, -CH₂-OH, -CH₂-O-*tert*-Butyl, -COOH or COO-tert-Butyl.

### R₅

According to the present invention, the carbohydrate building block (R₁-A-R₃) according to the above formulas, i.e. formulas 1(b) and 1(c), contain an R₅-group which is, individually, either hydrogen, methyl, benzyl, alkyl or substituted benzyl.

Preferably, R₅ is methyl (-CH₃).

### R₆

According to the present invention, the carbohydrate building block (R₁-A-R₃) according to the above formulas, i.e. formulas 1(b) and 1(c), contain a R₆-group which is, individually, selected among hydrogen, hydroxyl, protected hydroxyl, amine, or protected amine.

Preferably, R₆ is hydroxyl, protected hydroxyl or protected amine (e.g. -NHAc).

*Carbohydrate building blocks according to the invention containing an amino group or an activated carbonyl group linked via an amide bond (the -NHR₁ group) to the carbohydrate structure:*

In a highly preferred aspect the invention relates to the carbohydrate building block (R₁-A-R₃), according to one of the above formulas 1(a), 1(b) or 1(c), wherein R₁ together with the part of the carbohydrate structure (A) to which it is attached (the -NHR₁ group) form a free amine or a protected amine that is capable of forming amide bonds to a carbonyl group, and wherein R₃ together with the part of the carbohydrate structure (A) to which it is attached (the -COR₃ group) form a carboxylic acid or carboxylic acid ester group, that is capable of forming amide bonds with a free amine.

Such carbohydrate building blocks may be used to attach the carbohydrate structure to a molecular structure comprising a carbonyl group (e.g. a fatty acid residue) and/or a molecular structure comprising a free amine (e.g. an amino acid).

*Carbohydrate building blocks according to the invention containing a group linked via an amide bond (the -NHR₁ group) to the carbohydrate structure:*
When R₁ is a molecular group linked via an amide bond to the carbohydrate structure or the amino acid containing carbohydrate structure, the invention constitute a building block that is capable of attaching that group (and the carbohydrate structure) to a free amine (through the R₃-group which is either an OH group or a leaving group which, when reacted with a free amine, facilitates the formation of an amide bond between the carbonyl group and a free amine).

Preferably, such building blocks comprises one or more further groups B, C, D or A, wherein the groups B, C and D are as defined below and wherein A is as defined above.

Thus in a preferred aspect, the invention relates to a carbohydrate building block (R₁-A-R₃) according to the above formulas, i.e. formulas 1(a), 1(b) and 1(c), where R₁ is a group comprising one or more further groups B, C, D or A.

### B

According to the present invention B is a fatty acid residue such as e.g. tetradecanoic acid, hexadecanoic acid, octadecanoic acid, eicosanoic acid, docosanoic acid, tetracosanoic acid, tetradecanoic diacid, hexadecanoic diacid, octadecanoic diacid, eicosanoic diacid, docosanoic diacid or tetracosanoic diacid or analogs thereof.

### C

According to the present invention, C is an optional spacer structure, which may be used to separate the carbohydrate building block from other structures, such as a fatty acid residue (B), being part of the R₁ structure.

In a preferred aspect of this part of the invention, the optional C is either 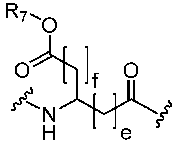 or 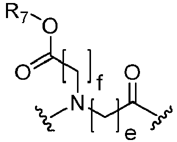 , wherein e is selected from the group consisting of 0, 1, and 2 and f is selected from the group consisting of 0, 1 and 2, and where R₇ either hydrogen or a protecting group (e.g. -*O*-benzyl, -*O*-Si(CH₃)₃ or *-O-tert*-butyl)*.*

### D

D is one or more amino acid residues. When part of the R₁ group, D may be one or more amino acids providing hydrophilicity and be part of a larger molecule.

In a preferred aspect of the present invention D provides additional hydrophilicity and provides spacing between other groups. Thus, in a preferred aspect of the invention, D is selected among Asp, Glu, Ser, Thr, Lys, Arg, Gly, Ala, Ornithine, Citrulline, 8-amino-3,6-dioxaoctanoic acid and 12-amino-4,7,10-trioxaoctadecanoic acid. This is particularly relevant when D is part of the R₁ group. Accordingly, in one aspect of the invention, when D is part of the R₁ group, D is an amino acid or a peptide comprising an amino acid selected from the group comprising Asp, Glu, Ser, Thr, Lys, Arg, Gly, Ala, Ornithine, Citrulline, 8-amino-3,6-dioxaoctanoic acid or 12-amino-4,7,10-trioxaoctadecanoic acid.

Such carbohydrate building blocks may be used to attach the carbohydrate structure containing a molecular structure attached via an amide linkage (e.g. a fatty acid residue) to a molecular structure comprising an amino acid, either the *N*-terminal amine or a side-chain amine (such as the epsilon amine of Lys).

Illustrative examples of such building block could be the following compounds:

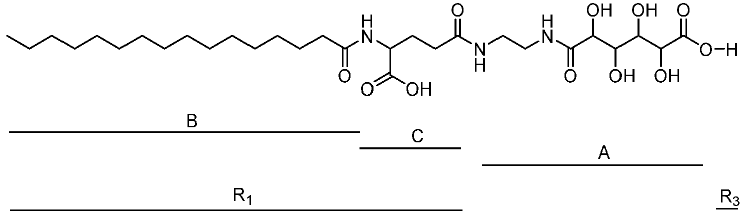

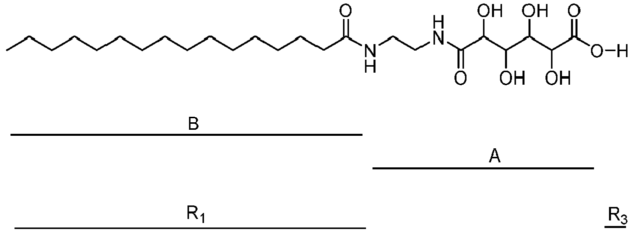

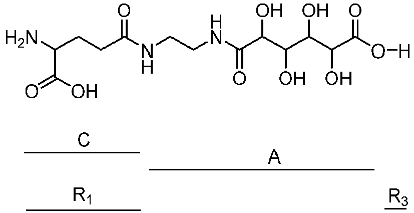

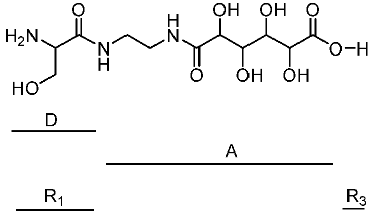

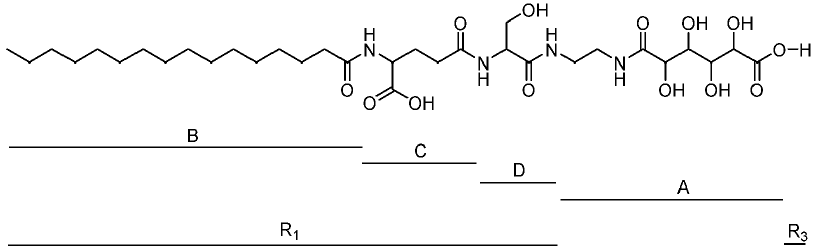

In the above illustrative examples the group underlined as "A" is shown as A according to the formula 1(a) of the invention. However, it should be understood as also disclosing similar illustrative examples where "A" is understood as A according to the formula 1(b) or 1(c) of the invention.

*Carbohydrate building blocks according to the invention containing a group linked via an amide bond (the -COR₃ group) to the carbohydrate structure:*

When R₃ is a molecular group linked via an amide bond to the carbohydrate structure, the invention constitutes a building block that is capable of attaching that group (and the carbohydrate structure) to a carbonyl group (through the R₁-group which is a group which, when reacted with a carbonyl group, facilitates the formation of an amide bond between the free amine and the carbonyl group).

When part of the R₃ group, D may be an amino acid or an amino acid that is part of a larger molecule such as a peptide or a protein.

Preferably, when the R₃ group is a single amino acid, a peptide or a protein, the amino acid or the part of the peptide or protein that forms linkage to the carbohydrate structure (A) is to be defined as D, and D is then preferably either a proteogenic amino acid attached via its *N*-terminal part to the carbohydrate structure, or D is an amino acid such as Lysine or cysteine attached via its side-chain to the carbohydrate structure, lysine being particularly preferred.

Such carbohydrate building blocks may be used to attach the carbohydrate structure containing a molecular structure attached via an amide linkage (e.g. one or more amino acid residue) to a molecular structure comprising a carbonyl group (e.g. a fatty acid residue), preferably comprising one or more of the groups B, C, D or A, wherein the groups A, B, C and D are as defined above.

*Agents comprising the carbohydrate building blocks according to the invention containing a group linked via an amide bond (the -NHR₁ group) to the carbohydrate structure and a group linked via an amide bond (the -COR₃ group) to the carbohydrate structure:*

When both R₁ and R₃ are molecular groups linked via an amide bond to the carbohydrate structure, the invention constitute an agent that has the combined functionalities of a carbohydrate structure and one or more additional structures, such as a fatty acid and or an amino acid residue.

Illustrative examples of such building block could be the following compounds:

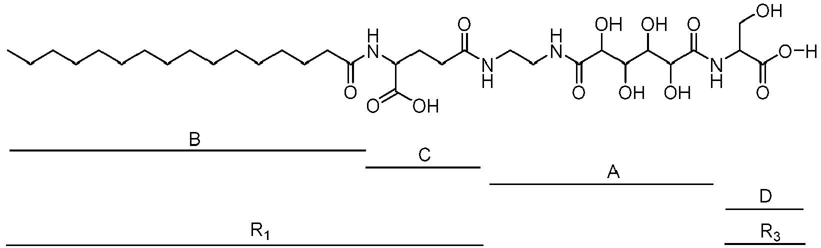

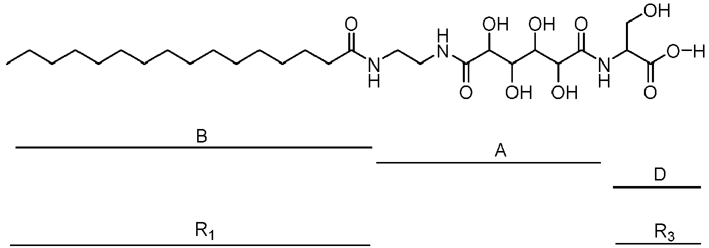

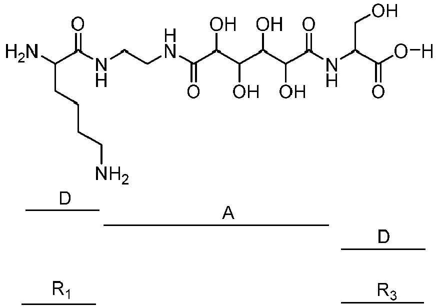

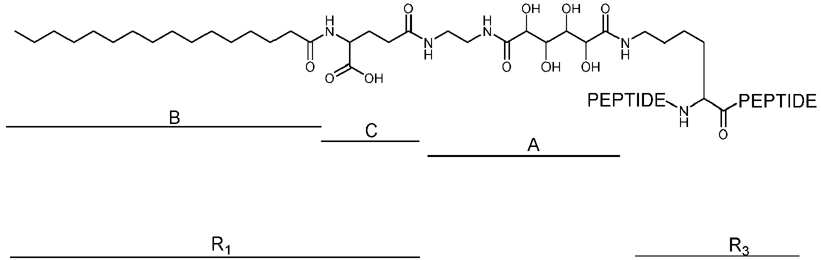

Thus, in a preferred aspect of the invention, the invention relates to an agent of the formula R₁-A-R₃, wherein A is a carbohydrate derivative and the agent R₁-A-R₃ is either 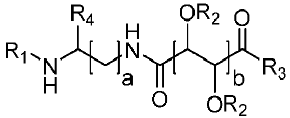 or 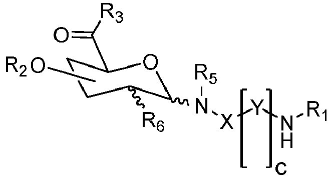 or 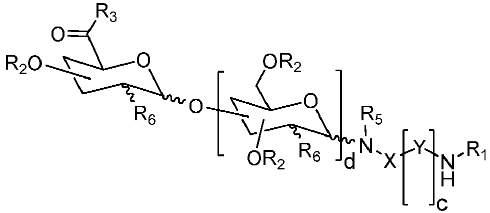 , wherein R₁ is hydrogen or an amino protecting group, or wherein R₁ is covalently linked via amide bonds to A, and R₁ is a group comprising one or more further groups attached via amide linkage,
each R₂ is, individually, either hydrogen or a hydroxyl protecting group,
a is 1, 2, 3, 4, 5 or 6,
b is 1, 2, 3 or 4,
c is 1, 2, 3, 4, 5 or 6,
d is 1, 2, 3, 4, 5, or 6, and
R₃ is either a hydroxyl, a protecting group (i.e. -*O*-benzyl, -*O*-Si(CH₃)₃ or *-O-tert-*butyl) or a leaving group (preferably -*O*-NHS or -*O*-Pfp) or wherein R₃ is one or more amino acids (D),
R₄ is either hydrogen, a hydroxyalkyl with 1-6 carbon atoms (-(CH₂)ₙOH, n is 1-6), a protected hydroxyalkyl with 1-6 carbon atoms (-(CH₂)ₙOR₂, n is 1-6), an alkyl carboxylic acid with 1-6 carbon atoms (-(CH₂)ₙCOOR₇, n is 1-6 and R₇ is either hydrogen or a protecting group (e.g. -*O*-benzyl, -*O*-Si(CH₃)₃ or *-O-tert*-butyl)) or a protected alkyl carboxylic acid of 1-6 carbon atoms (-(CH₂)ₙCOOR₇, n is 1-6 and R₇ either hydrogen or a protecting group (e.g. -*O*-benzyl, -*O*-Si(CH₃)₃ or *-O-tert*-butyl))*.*
R₅ is hydrogen, methyl, benzyl, alkyl or substituted benzyl.
R₆ is hydrogen, hydroxyl, protected hydroxyl, amine or protected amine (such as -NHAc),
X is oxygen or nitrogen (NH), and
Y is either -CH₂- or -(CH₂CH₂O)ₙCH₂CH₂-, where n is selected from the group consisting of 1, 2, 3, 4, or 5.

In a highly preferred aspect of the invention, R₁ is hydrogen or an amino protecting group.

In another highly preferred aspect, R₁ is a molecular group linked via an amide bond to the carbohydrate structure or the amino acid containing carbohydrate structure.

Preferably, R₁ is a molecular structure comprising one or more of the groups B, C, D or A, where
A is carbohydrate derivative as defined above,
B is a fatty acid residue as defined above,
C is either 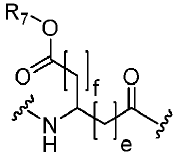 or 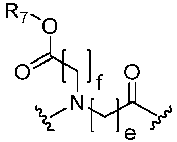 , wherein e is selected from the group consisting of 0, 1, and 2 and f is selected from the group consisting of 0, 1 and 2, and where R₇ either hydrogen or a protecting group (e.g. -*O*-benzyl, -*O*-Si(CH₃)₃ or *-O-tert-*butyl), and
D is one or more amino acid residues as defined above.

In a highly preferred aspect of the invention, R₁ is a group comprising a fatty acid residue B, wherein B is 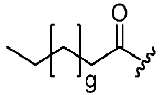 or 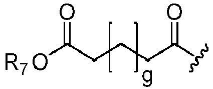 wherein, g is an integer of from 10 - 20 , and where R₇ is either hydrogen or a protecting group (e.g. -*O*-benzyl, -*O*-Si(CH₃)₃ or *-O-tert*-butyl)*.* Preferably, g is and even number, i.e. 10, 12, 14, 16, 18 or 20.

In another highly preferred aspect of the invention, R₃ is hydroxyl or a leaving group (e.g. -*O*-NHS or -*O*-Pfp).

In another highly preferred aspect of the invention, R₃ is one or more amino acids (D). The amino acid(s) (D) may be part in a peptide or protein structure.

In a preferred aspect of the invention, D is an amino acid, such as Asp, Glu, Ser, Thr, Lys, Arg, Gly, Ala, Ornithine, Citrulline, 8-amino-3,6-dioxaoctanoic acid or 12-amino-4,7,10-trioxaoctadecanoic acid.

In another highly preferred embodiment D is a proteogenic amino acid attached via its *N*-terminal part to the carbohydrate structure, or D is an amino acid such as Lysine or cysteine, lysine being particularly preferred.

In a highly preferred aspect of the invention, R₁ is hydrogen or an amino protecting group, and R₃ is hydroxyl or a leaving group (e.g. -*O*-NHS or -*O*-Pfp).

In a highly preferred aspect of the invention, R₁ is a molecular group comprising a fatty acid residue (B).

In a highly preferred aspect of the invention, R₁ is a molecular group comprising a fatty acid residue (B), and R₃ is hydroxyl or a leaving group (e.g. -*O*-NHS or -*O*-Pfp).

In a highly preferred aspect of the invention, R₁ is hydrogen or an amino protecting group, and R₃ is one or more amino acids (D).

In a highly preferred aspect, the invention relates to agents comprising the carbohydrate building block A according to the invention and one or more of the structures B, C and D as defined above.

Preferred examples of agents are the agents

B-C-E-R₃,

or

B-E-R₃,

where R₃, A, B, C and D are as defined above, and where E is A or where E consist of A and D. Illustrative examples hereof are sequences such as BCA-R₃, BCAD-R₃, BAD-R₃, BCDAD-R₃, BCDADAAAD-R₃.

In a highly preferred aspect of the invention the R₃ group is a peptide or a protein.

In a highly preferred aspect of the invention the R₃ group is a peptide or a protein, and the R₁-group comprises a fatty acid residue. Such peptides or proteins may constitute molecules combining the functionalities of the peptide or protein, and the carbohydrate and the fatty acid part of the molecule. Such molecules may combine a therapeutic effect with solubility and increased stability.

Thus, the invention also relates to agents according to the invention for use as medicaments, and for the use of the agents according to the invention as medicaments, and for the use of the agents according to the invention for the manufacture of a medicament.

The invention also relates to the use of the agents according to the invention for the manufacture of an agent capable of attaching fatty acids on one or more amino groups in a peptide or protein.

### Examples

Unless otherwise stated, reagents used throughout the examples were purchased from Sigma-Aldrich (Denmark).

### Example 1: Synthesis of (2S,3R,4S,5R)-6-((2-(N-Fmoc)aminoethyl)amino)-2,3,4,5-tetraacetoxy-6-oxohexanoic acid

### Step a). Synthesis of mucic acid tetraacetate

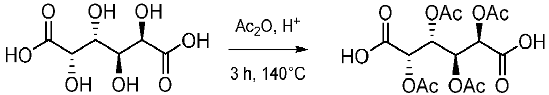

Mucic acid (20 g, 95.3 mmol) was added to a stirred solution of acetic anhydride (150 ml) and conc. sulphuric acid (10 drops). The reaction mixture was maintained at 140°C for 3 hours and then allowed to cool to room temperature. Cold water (200 ml) was added to the reaction mixture, and the precipitate was collected by filtration and dried *in vacuo* overnight to provide the pure product as a white solid. Yield: 16.75 g (46%). 1H-NMR (300 MHz, MeOD) δ 5.70 (s, 2H), 5.15 (s, 2H), 2.15 (s, 6H), 2.05 (s, 6H). 13C-NMR (75 MHz, MeOD) δ 171.65, 170.55, 170.12, 70.74, 69.60, 20.40, 20.32.

Step b). Synthesis of (2S,3R,4S,5R)-6-((2-(N-Fmoc)aminoethyl)amino)-2,3,4,5-tetraacetoxy-6-oxohexanoic acid (Alternative name: N-Fmoc-AE-tetrahydroxy-hexanoic acid).

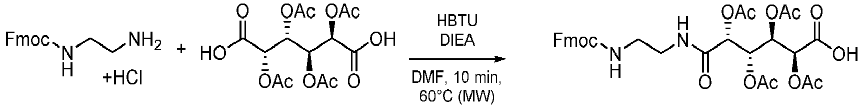

In a Biotage Initiator vial (2-5 ml), Fmoc-1,2-ethylenediamine*HCl (100 mg, 0.31 mmol, purchased from Iris Biotech GmbH (Marktredwitz Germany)), mucic acid tetraacetate (118.6 mg, 0.31 mmol), *N*-[(1H-benzotriazol-1-yl)-(dimethylamino)methylene]-N-methylmethanaminium hexafluoro phosphate *N*-oxide (HBTU) (119 mg, 0.31 mmol purchased from GL Biochem Ltd. (Shanghai, China)), and *N,N-*diisopropylethylamine (DIEA) (0.08 ml , 0.47 mmol) was dissolved in *N,N-*dimethylformamide (DMF) (3 ml). The vial was sealed and heated to 60°C for 10 min with magnetic stirring. The crude reaction mixture was concentrated using the Biotage V10 evaporator. Crude compound was dissolved in acetonitrile (9.5 ml) and acetic acid (0.5 ml), filtered through a syringe filter (0.45 µm) and purified by preparative RP-HPLC in two steps using a Waters 150 LC system.

In the first purification step, crude reaction mixture was purified using a preparative Daiso C18 column (10 µm, 200Å, 50 x 250 mm) and a solvent system containing solvent A (0.1% TFA in H₂O) and solvent B (0.1% TFA in acetonitrile). A 10-100% linear gradient (0-27 min: 10-100%; 27-33 min: 100%; flow rate: 45 ml/min) of solvent B was applied as eluent. The column eluate was monitored by UV absorbance at 215 nm and 254 nm. The product-containing fractions were quantified by UPLC-MS using a Waters Xevo TQ system equipped with an analytical Acquity UPLC BEH C18 column (1.7µ, 100 x 2.1 mm, Waters). Fractions were pooled and lyophilized to provide the product in 75% crude yield (as determined by UPLC-MS).

In the second purification step, the lyophilized fractions were re-dissolved in H₂O/acetonitrile (50:50, v/v) and purified using a preparative Gemini-NX C18 column (5µ, 110Å, 30 x 100 mm, Phenomenex) and a solvent system containing solvent A (0.1% TFA in H₂O) and solvent B (0.1% TFA in acetonitrile). A 10-100% linear gradient (0-27 min: 10-100%; 27-33 min: 100%; flow rate: 30 ml/min) of solvent B was applied as eluent. The column eluate was monitored by UV absorbance at 215 nm and 254 nm. The product-containing fractions were quantified by UPLC-MS (as above).

Fractions were pooled and lyophilized to provide the pure product as a white powder. Yield: 21.3 mg (11%). 1H-NMR (300 MHz, CD₃CN) δ 7.83 (d, *J* = 7.3 Hz, 2H), 7.65 (d, *J* = 7.5 Hz, 2H), 7.42 (t, *J* = 7.5 Hz, 2H), 7.34 (t, *J* = 7.4 Hz, 2H), 7.08 (s, 1H), 5.78 (s, 1H), 5.56 (s, 2H), 5.14 (s, 1H), 5.06 (s, 1H), 4.34 (d, *J* = 7.1 Hz, 2H), 4.23 (t, *J* = 6.4 Hz, 1H), 3.35-2.99 (m, 4H), 2.14 (s, 3H), 2.08 (s, 3H), 1.99 (s, 3H), 1.97 (s, 3H). 13C-NMR (75 MHz, CD₃CN) δ 170.83, 168.56, 167.85, 145.15, 142.10, 128.66, 128.09 , 126.11, 120.94, 71.87, 70.40, 69.40 , 68.85, 67.10, 48.04, 40.92, 40.48, 21.00, 20.67, 20.60. ESI-MS m/z calculated for C₃₁H₃₅N₂O₁₃ (M+H)⁺ 643.21, found 643.07.

### Example 2: Synthesis of N-[N-methyl-O-(2-Fmoc-aminoethyl)-hydroxylamino] β-D Glucopyranuronic acid:

### Step a): Synthesis of Boc-N-methyl hydroxylamine

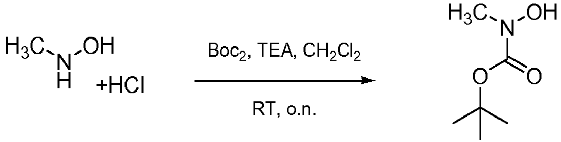

*N*-methyl hydroxylamine*HCl (3.34 g, 40 mmol) and di-tert-butyl dicarbonate (7.35 ml, 32 mmol) was dissolved in ice-cooled anhydrous dichloromethane (DCM) (250 ml). Triethylamine (TEA) (12.03 ml, 80 mmol) was added under cooling, and the resulting mixture was stirred overnight at room temperature under argon. Reaction volume was reduced to 50% using rotary evaporation, and ethyl acetate (200 ml) was added. The mixture was washed with 5% aq. acetic acid (v/v, 2 x 200 ml), and brine (2 x 200 ml). The organic layer was dried over MgSO₄ and filtered. The solvent was removed *in vacuo* to yield a clear pinkish oil. Crude reaction mixture was purified by flash chromatography using the Biotage Isolera with a SNAP HP-Sil 100 g column as stationary phase and heptane/ethyl acetate as mobile phase (1 CV: 10%; 10 CV: 10-100%; 2 CV: 100%, flow rate: 50 ml/min). Column eluate was monitored in real-time using the Biotage Dalton MS unit. Correct fractions were pooled, concentrated and dried *in vacuo* to yield the product as a colorless liquid. Yield: 2.26 g (38%). 1H NMR (300 MHz, CDCl₃) δ 3.08 (s, 3H), 1.39 (s, 9H). 13C-NMR (75 MHz, CDCl₃) δ 157.71, 81.51, 38.12, 28.26. ESI-MS m/z calculated for C₆H₁₄NO₃ (M+H)⁺ 148.09, found 148.0.

### Step b) Synthesis of 2-(N-Boc-N-methylaminooxy)-N-Boc-ethanamine

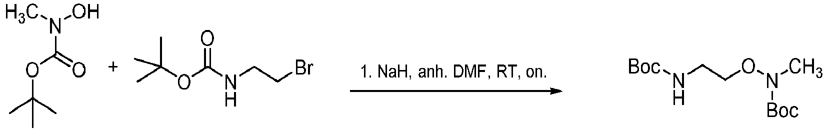

Boc-*N*-methyl hydroxylamine and NaH was dissolved in anhydrous *N*,*N*-dimethylformamide (DMF) (45 ml) with activated molecular sieves (4Å) using magnetic stirring for 30 min. *N*-Boc-2-bromoethyl-amine (N-Boc-2-bromoethane-1-amine was synthesized in-house by standard techniques, but may alternatively be purchased from Sigma-Aldrich (prod. no 17354)) was added, and the resulting mixture was stirred overnight at room temperature under argon. Crude reaction mixture was concentrated using the Biotage V10 and purified by flash chromatography using the Biotage Isolera One with a SNAP HP-Sil 50 g column as stationary phase and heptane/ethyl acetate as mobile phase (3 CV: 10%; 10 CV: 10-50%; 2 CV: 50%, flow rate: 50 ml/min). Column eluate was monitored in real-time using the Biotage Dalton MS unit (m/z 290.0, 312.6, 602.8). Correct fractions were pooled, concentrated and dried *in vacuo* to yield the Boc-protected intermediate as a clear colorless oil. Yield: 992 mg (54%). 1H-NMR (300 MHz, CDCl₃) δ 5.41 (s, 1H), 3.85 (d, J = 4.9 Hz, 2H), 3.33 (d, J = 5.0 Hz, 2H), 3.07 (s, 3H), 1.49 (s, 9H), 1.44 (s, 9H).

### Step c: Synthesis of 2-(methylaminooxy)ethanamine

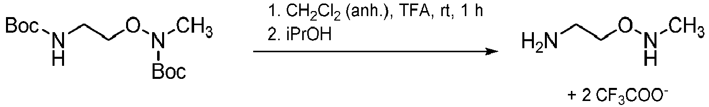

2-(*N*-Boc-*N*-methylaminooxy)-*N*-Boc-ethanamine (370 mg, 1.3 mmol) was dissolved in anhydrous dichloromethane (DCM) (1 ml), and trifluoroacetic acid (TFA, purchased from Iris Biotech GmbH (Germany)) (1 ml) was added. The reaction mixture was stirred overnight at room temperature under argon. Cold 2-propanol (*i*PrOH) (25 ml) was added to quench the reaction, and the solvent was removed *in vacuo* to yield the product as TFA salt. Yield: 330 mg (83%). 1H-NMR (300 MHz, D₂O) δ 4.45 (d, J = 5.0 Hz, 2H), 3.42 (d, J = 5.0 Hz, 2H), 3.10 (s, 3H).

### Step d): Synthesis of N-[N-methyl-O-(2-Fmoc-aminoethyl)-hydroxylamino] β-D glucopyranuronic acid.

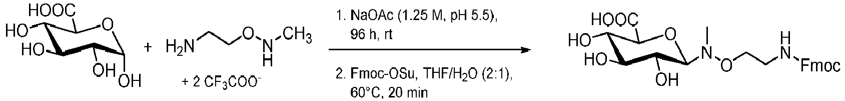

D-Glucuronic acid (15.5 mg, 0.08 mmol, Sigma-Aldrich) and 2-(methylaminooxy)ethanamine (126.5 mg, 0.4 mmol) was dissolved in sodium acetate (NaOAc) buffer (1.25 M, pH 5.5, final concentrations 50 mM and 250 mM, respectively) and stirred for 96 hours at room temperature. The pH of the reaction mixture was adjusted by addition of Na₂CO₃. 9-Fluorenylmethyl N-succinimidyl carbonate (Fmoc-OSu, 161.9 mg, 0.48 mmol) dissolved in tetrahydrofuran (THF, 3 ml) was added, and the resulting mixture was stirred at 60° C for 20 min. THF was removed *in vacuo,* and the crude compound was lyophilized, re-dissolved in water and filtered through a syringe filter (0.45 µm). The compound was purified by RP-HPLC (Dionex Ultimate 3000 system) on a preparative C18 column (5 µm, 300 Å, 250 mm x 21.2 mm, Phenomenex) using the following solvent system: solvent A, water; solvent B, acetonitrile. B gradient elution (0-28 min: 10 to 100%) was applied at a flow rate of 15 ml/min and column effluent was monitored by UV absorbance at 302 nm (RT = 18.5 min). The product-containing fractions were pooled and lyophilized to yield the target compound (>90% purity by analytical HPLC). Identification was carried out by ESI-MS (MSQ Plus Mass spectrometer, Thermo). ESI-MS m/z calculated for C₂₄H₂₉N₂O₉ (M+H)⁺ 489.5, found 489.2.

Agent of formula 1(c) can be synthesized by methods comparable to the above (step d)), by substituting the D-glucuronic acid used above in step d) with a desired carbohydrate obtained by linking D-glucuronic acid to another carbohydrate(s) by standard methods. The 2-(methylaminooxy)ethanamine can be attached and the free amine can be protected by the same procedure as in step d), at the anomeric carbon of the carbohydrate.

### Example 3: Acylation of Arg³⁴-GLP1(7-37)-OH using (2S,3R,4S,5R)-6-((2-(N-Fmoc)aminoethyl)amino)-2,3,4,5-tetraacetoxy-6-oxohexanoic acid (Alternative name: N-Fmoc-AE-tetrahydroxy-hexanoic acid) produced in example 1.

### Synthesis of Arg³⁴Lys²⁶(Pam-γGlu-(AE-tetrahydroxy-hexanoyl))-GLP1(7-37)-OH

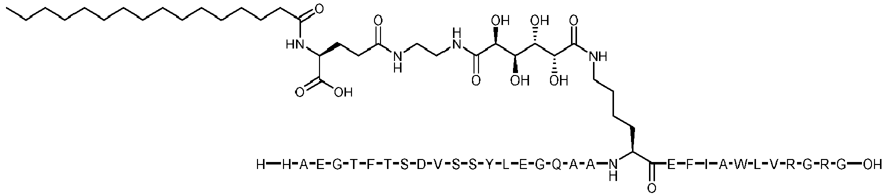

Arg³⁴Lys²⁶(Pam-γGlu-((2R,3S,4R,5S)-6-((2-aminoethyl)amino)-2,3,4,5-tetrahydroxy-6-oxohexanoyl)-)-GLP1(7-37)-OH was synthesized on Tenta-Gel S PHB resin (0.27 mmol/g, Rapp Polymere GmbH) using the Fmoc/tBu strategy by automated solid-phase peptide synthesis (SPPS) on the SyroII Peptide Synthesizer (MultiSynTech/Biotage).

### Step a): Peptide synthesis

The couplings were performed using 9-fluorenylmethyloxycarbonyl (Fmoc) *N^{α}*-protected amino acids. The side-chain protecting groups used were tert-butyl (tBu, for Ser, Thr, Tyr), 2,2,4,6,7-pentamethyl-dihydrobenzofuran-5-sulfonyl (Pbf, for Arg), trityl (Trt, for Asn, Gln, His, Cys), and tert-butyloxycarbonyl (Boc, for Trp).

Residues six and seven was incorporated using Fmoc-L-Phe-L-Thr[PSI(Me,Me)Pro]-OH. All *N^{α}-*Fmoc-protected amino acids and Boc-His(Trt)-OH (4 eq, Iris Biotech GmbH) were coupled using *N*,*N*'-diisopropylcarbodiimide (DIC) (3.8 eq, Iris Biotech GmbH) as coupling reagent, ethyl cyanoglyoxylate-2-oxime (Oxyma) (4 eq, Iris Biotech GmbH) as additive, and *N*,*N*-dimethylformamide (DMF) as solvent. Coupling times were 2 x 2 hr at room temperature, except for the coupling with Fmoc-L-Phe-L-Thr[PSI(Me,Me)Pro]-OH, which was performed as a 4 hr single coupling. *N*^{α}-deprotection was performed at room temperature for 3 min using 40% piperidine in *N*-methyl-2-pyrrolidinone (NMP) followed by 22 min using 20% piperidine in NMP.

Loading of the C-terminal residue (Gly) was achieved by anhydride formation. Fmoc-Gly-OH (10 eq), DIC (5 eq) and 1-methylimidazole (MeIm, 5 eq, Sigma-Aldrich) were dissolved in a mixture of DCM and DMF (80/20, v/v). The mixture was added to the resin, and the resulting slurry was stirred for 2 hr at room temperature. The resin was transferred to a fritted syringe, excess reagents were removed by suction filtration, and the resin was washed with DCM and NMP. The entire loading procedure was repeated, and the resin was dried *in vacuo.* The resin loading was calculated to 0.25 mmol/g according the procedure described by Chan & White (WC Chan & PD White. *Fmoc Solid Phase Peptide Synthesis - A Practical Approach.* Oxford University Press 2000).

To obtain site selective incorporation on the *N*^{ε}-Lys, the Lys residue was side-chain protected using the alloc (All) protecting group, and a *N^{α}*-Boc-protected His (Boc-His(Trt)-OH, Iris Biotech GmbH) was incorporated as the *N*-terminal amino acid.

### Step b: Production of Arg³⁴Lys²⁶(2R,3S,4R,5S)-6-((2-aminoethyl)amino)-2,3,4,5-tetrahydroxy-6-oxohexanoyl)-)-GLP1(7-37)-OH

On-resin installation of the hydrophilic spacer and fatty acid moiety was performed on the *N^{ε}*-amine of the side-chain of the available Lys residue. Following linear assembly of the peptide backbone, the alloc protecting group was removed using tetrakis(triphenylphosphine)palladium(0) (Pd(PPh₃)₄) and borane dimethylamine complex as scavenger. The resin was washed three times with dichloromethane (DCM) and finally suspended in anhydrous DCM. Oxygen was removed from the resin-DCM mixture by purging with nitrogen flow for 30 min and borane dimethylamine complex (1.5 eq) was added. The mixture was stirred for 5 min under nitrogen flow followed by addition of Pd(PPh₃)₄ (1.0 eq). The mixture reacted for 2 hr at room temperature. After Alloc removal, the resin was washed using 3 x DCM, 3 x NMP, 3 x piperidine-DMF (1:4), 2 x NMP, 1 x DCM, and 2 x NMP.

### Carbohydrate (component A)

The *N^{ε}*-acylation was performed by coupling (2S,3R,4S,5R)-6-((2-(*N-*Fmoc)aminoethyl)amino)-2,3,4,5-tetraacetoxy-6-oxohexanoic acid (2 eq) to the free amine using *N*-[(dimethylamino)-1*H*-1,2,3-triazolo[4,5-*b*]pyridin-1-ylmethylene]-*N-*methylmethanaminium hexafluorophosphate *N*-oxide (HATU, GL Biochem Shanghai) (1.9 eq) as coupling reagent, DIEA (3.8 eq, Iris Biotech GmbH) as base and DMF as solvent for 16 hr at room temperature.

### Spacer (component C)

The Fmoc group was removed as for standard SPPS, and Fmoc-Glu-OtBu (GL Biochem Shanghai, 4 eq) was coupled using (HATU) (3.8 eq) DIEA (7.6 eq) in DMF for 4 hr at room temperature.

### Fatty acid (component B)

The Fmoc group was removed as for standard SPPS, and palmitic acid (Pam, hexadecanoic acid) (4 eq) was incorporated using HATU (3.8 eq) and DIEA (7.6 eq) for 16 hr at room temperature.

### Product deprotection and release

Removal of the acetyl protecting groups was performed by washing the resin with methanol, followed by two treatments with sodium methoxide (NaOMe) solution (0.5 M in methanol) for 20 min at room temperature. After de-acylation, the resin was washed with 3 x methanol, 3 x NMP, 3 x dichloromethane, 3 x NMP, and 3 x dichloromethane. Finally, the product was simultaneously side-chain de-protected and released from the solid support by a trifluoroacetic acid (TFA) cocktail containing triethyl silane (TES) and H₂O (95/2.5/2.5, v/v, 5 ml) as scavengers for 2 hr. The peptide was precipitated by the addition of cold diethyl ether to yield the crude peptide as white powder.

### Product purification

The peptide was purified by RP-HPLC (Dionex Ultimate 3000 system) on a preparative column (Gemini C18 column, 5 µm, 110 Å, 100 mm x 21.2 mm, Phenomenex Inc.) by a two-step process using the following solvent system: solvent A, water containing 0.1% TFA; solvent B, acetonitrile containing 0.1% TFA. In the first step purification step, B gradient elution (0-27 min: 10 to 70%) was applied at a flow rate of 15 ml/min and column effluent was monitored by UV absorbance at 215 and 254 nm (RT = 18.5 min). The product-containing fractions were pooled and lyophilized, followed by the second purification step, in which B gradient elution (0-27 min: 45 to 55%) was applied at a flow rate of 15 ml/min and column effluent was monitored by UV absorbance at 215 and 254 nm (RT = 3.55 min). Identification was carried out by ESI-MS (MSQ Plus Mass spectrometer, Thermo). MilliQ (Millipore) water was used for RP-HPLC analyses and purifications.

The peptide was quantified by analytical HPLC (Dionex Ultimate 3000 system equipped with a DAD detector) on an analytical column (Gemini NX C18 column, 3 µm, 110 Å, 50 mm x 4.6 mm, Phenomenex Inc.) using the following solvent system: solvent A, water containing 0.1% HCOOH; solvent B acetonitrile containing 0.1% HCOOH. B gradient elution (0-1 min: 5%; 1-10 min: 5 to 100%) was applied at a flow rate of 1.0 ml/min. MilliQ water was used for analytical HPLC analysis. The final product were obtained with >95% purity. ESI-MS m/z calculated for C₁₈₀H₂₈₂N₄₅O₅₇ (M+3H)³⁺ 1329.5, found 1329.2. ESI-MS m/z calculated for C₁₈₀H₂₈₁N₄₅O₅₇ (M+2H)²⁺ 997.4, found 997.0.

## Claims

1. An agent of the formula R₁-A-R₃, wherein A is a carbohydrate derivative and the agent R₁-A-R₃ is either 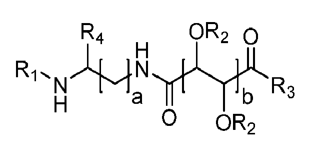 or 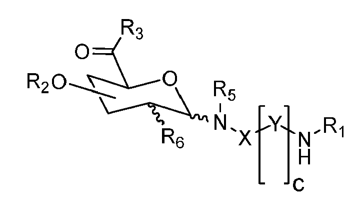 or 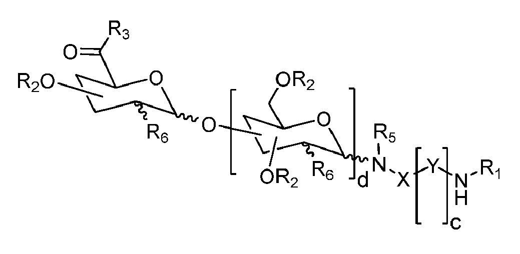 , wherein R₁ is hydrogen or an amino protecting group, or
wherein R₁ is a group comprising one or more further groups attached via amide linkage to A,
each R₂ is, individually, either hydrogen or a hydroxyl protecting group,
a is 1, 2, 3, 4, 5 or 6,
b is 1, 2, 3 or 4,
c is 1, 2, 3, 4, 5 or 6,
d is 1, 2, 3, 4, 5, or 6, and
R₃ is either hydroxyl, a protecting group or a leaving group or wherein R₃ is one or more amino acids (D),
R₄ is either hydrogen, a hydroxyalkyl with 1-6 carbon atoms, a protected hydroxyalkyl with 1-6 carbon atoms, an alkyl carboxylic acid with 1-6 carbon atoms or a protected alkyl carboxylic acid of 1-6 carbon atoms,
R₅ is hydrogen, methyl, benzyl, alkyl or substituted benzyl,
R₆ is hydrogen, hydroxyl, protected hydroxyl, amine or protected amine,
X is oxygen or nitrogen (NH), and
Y is either -CH₂- or -(CH₂CH₂O)ₙCH₂CH₂-, where n is selected from the group consisting of 1, 2, 3, 4, or 5.

2. Agent according to claim 1 wherein R₁ is hydrogen or an amino protecting group.

3. Agent according to claim 1 wherein R₁ is covalently linked via amide bonds to A, and wherein R₁ is a group comprising one or more further groups B, C, D or A where
A is carbohydrate derivative as defined in claim 1,
B is a fatty acid residue,
C is either 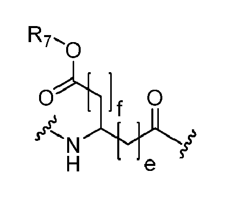 or 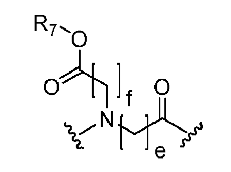 , wherein e is selected from the group consisting of 0, 1, and 2 and f is selected from the group consisting of 0, 1 and 2, and where R₇ is either hydrogen or a protecting group, and
D is one or more amino acid residues as defined above.

4. Agent according to claim 3, wherein R₁ is a group comprising a fatty acid residue B, wherein B is 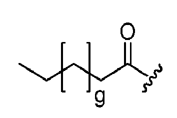 or 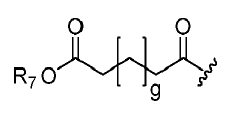 wherein g is an integer of from 10 - 20, and R₇ is either hydrogen or a protecting group.

5. Agent according to any of claims 1 - 4 wherein R₃ is hydroxyl or a leaving group.

6. Agent according to any of claims 1 - 4 wherein R₃ is one or more amino acids (D) as defined above.

7. Agent according to claims 2 and 5.

8. Agent according to claims 2 and 6.

9. Agent according to any of claims 1, 3, 4, 5 or 6, wherein R₁ comprises the group B.

10. Agent according to claims 5 and 9.

11. Agent according to claim 9, consisting of the formula
B-C-E-R₃,
or
B-E-R₃,
where A, B, C and D are as defined above, and where E is A or where E consist of one or more A and one or more D.

12. Agent according to any of the above claims 6, 8, 10 or 11 wherein the R₃ group is a peptide or a protein.

13. Agent according to any of the above claims 1 - 12 for use as a medicament or for use in the manufacture of a medicament.

14. Use of an agent according to any of claims 1 - 13 for the manufacture of an agent capable of lipidating one or more amino groups in a peptide or protein.

15. Use of an agent according to any of claims 1 - 14 for the lipidation of one or more amino groups in a peptide or protein.
